# EUROPEAN PATENT APPLICATION

(11) **EP 3 087 995 A1**
(43) Date of publication of application: **02.11.2016**
(21) Application number: 15165741.8
(22) Date of filing: 29.04.2015
(51) Int. Cl.: A61K 38/17, A61K 48/00

(54) **MUTANTS OF SRSF1 (ASF/SF2) FOR TREATING SPINAL MUSCULAR ATROPHY**

(71) Applicant: ETH Zurich, 8092 Zurich (CH)
(72) Inventor: Allain, Frédéric, 8046 Zürich (CH); Cléry, Antoine, 8046 Zürich (CH)

(57) **Abstract**

The invention relates to non-naturally occurring proteins with a splicing factor activity that are able to facilitate the inclusion of exon 7 of the SMN1 and/or SMN2 gene into the mature mRNA. The disclosed non-naturally occurring proteins share at least 70% sequence identity with hSRSF1 (ASF/SF2) and can be used in the treatment of spinal muscular atrophy.

SEQUENCE LISTING

<110>  ETH Zuerich

<120>  Mutants of SRSF1 (ASF/SF2) for treating Spinal Muscular Atrophy

<130>  2015-003

<160>  8

<170>  PatentIn version 3.5

<210>  1
<211>  248
<212>  PRT
<213>  Homo sapiens

<400>  1

Met Ser Gly Gly Gly Val Ile Arg Gly Pro Ala Gly Asn Asn Asp Cys
1               5                   10                  15

Arg Ile Tyr Val Gly Asn Leu Pro Pro Asp Ile Arg Thr Lys Asp Ile
            20                  25                  30

Glu Asp Val Phe Tyr Lys Tyr Gly Ala Ile Arg Asp Ile Asp Leu Lys
        35                  40                  45

Asn Arg Arg Gly Gly Pro Pro Phe Ala Phe Val Glu Phe Glu Asp Pro
    50                  55                  60

Arg Asp Ala Glu Asp Ala Val Tyr Gly Arg Asp Gly Tyr Asp Tyr Asp
65                  70                  75                  80

Gly Tyr Arg Leu Arg Val Glu Phe Pro Arg Ser Gly Arg Gly Thr Gly
                85                  90                  95

Arg Gly Gly Gly Gly Gly Gly Gly Gly Gly Ala Pro Arg Gly Arg Tyr
            100                 105                 110

Gly Pro Pro Ser Arg Arg Ser Glu Asn Arg Val Val Val Ser Gly Leu
        115                 120                 125

Pro Pro Ser Gly Ser Trp Gln Asp Leu Lys Asp His Met Arg Glu Ala
    130                 135                 140

Gly Asp Val Cys Tyr Ala Asp Val Tyr Arg Asp Gly Thr Gly Val Val
145                 150                 155                 160

Glu Phe Val Arg Lys Glu Asp Met Thr Tyr Ala Val Arg Lys Leu Asp
                165                 170                 175

Asn Thr Lys Phe Arg Ser His Glu Gly Glu Thr Ala Tyr Ile Arg Val
            180                 185                 190

Lys Val Asp Gly Pro Arg Ser Pro Ser Tyr Gly Arg Ser Arg Ser Arg
        195                 200                 205

Ser Arg Ser Arg Ser Arg Ser Arg Ser Arg Ser Asn Ser Arg Ser Arg
    210                 215                 220

Ser Tyr Ser Pro Arg Arg Ser Arg Gly Ser Pro Arg Tyr Ser Pro Arg
225                 230                 235                 240

His Ser Arg Ser Arg Ser Arg Thr
                245

<210>  2
<211>  248
<212>  PRT
<213>  Homo sapiens

<400>  2

Met Ser Gly Gly Gly Val Ile Arg Gly Pro Ala Gly Asn Asn Asp Cys
1               5                   10                  15

Arg Ile Tyr Val Gly Asn Leu Pro Pro Asp Ile Arg Thr Lys Asp Ile
            20                  25                  30

Glu Asp Val Phe Tyr Lys Tyr Gly Ala Ile Arg Asp Ile Asp Leu Lys
        35                  40                  45

Asn Arg Arg Gly Gly Pro Pro Phe Ala Phe Val Glu Phe Glu Asp Pro
    50                  55                  60

Arg Asp Ala Glu Asp Ala Val Tyr Gly Arg Asp Gly Tyr Asp Tyr Asp
65                  70                  75                  80

Gly Tyr Arg Leu Arg Val Asn Phe Pro Arg Ser Gly Arg Gly Thr Gly
                85                  90                  95

Arg Gly Gly Gly Gly Gly Gly Gly Gly Gly Ala Pro Arg Gly Arg Tyr
            100                 105                 110

Gly Pro Pro Ser Arg Arg Ser Glu Asn Arg Val Val Val Ser Gly Leu
        115                 120                 125

Pro Pro Ser Gly Ser Trp Gln Asp Leu Lys Asp His Met Arg Glu Ala
    130                 135                 140

Gly Asp Val Cys Tyr Ala Asp Val Tyr Arg Asp Gly Thr Gly Val Val
145                 150                 155                 160

Glu Phe Val Arg Lys Glu Asp Met Thr Tyr Ala Val Arg Lys Leu Asp
                165                 170                 175

Asn Thr Lys Phe Arg Ser His Glu Gly Glu Thr Ala Tyr Ile Arg Val
            180                 185                 190

Lys Val Asp Gly Pro Arg Ser Pro Ser Tyr Gly Arg Ser Arg Ser Arg
        195                 200                 205

Ser Arg Ser Arg Ser Arg Ser Arg Ser Arg Ser Asn Ser Arg Ser Arg
    210                 215                 220

Ser Tyr Ser Pro Arg Arg Ser Arg Gly Ser Pro Arg Tyr Ser Pro Arg
225                 230                 235                 240

His Ser Arg Ser Arg Ser Arg Thr
                245

<210>  3
<211>  248
<212>  PRT
<213>  Homo sapiens

<400>  3

Met Ser Gly Gly Gly Val Ile Arg Gly Pro Ala Gly Asn Asn Asp Cys
1               5                   10                  15

Arg Ile Tyr Val Gly Asn Leu Pro Pro Asp Ile Arg Thr Lys Asp Ile
            20                  25                  30

Glu Asp Val Phe Tyr Lys Tyr Gly Ala Ile Arg Asp Ile Asp Leu Lys
        35                  40                  45

Asn Arg Arg Gly Gly Pro Pro Phe Ala Phe Val Glu Phe Glu Asp Pro
    50                  55                  60

Arg Asp Ala Glu Asp Ala Val Tyr Gly Arg Asp Gly Tyr Asp Tyr Asp
65                  70                  75                  80

Gly Tyr Arg Leu Arg Val Lys Phe Pro Arg Ser Gly Arg Gly Thr Gly
                85                  90                  95

Arg Gly Gly Gly Gly Gly Gly Gly Gly Gly Ala Pro Arg Gly Arg Tyr
            100                 105                 110

Gly Pro Pro Ser Arg Arg Ser Glu Asn Arg Val Val Val Ser Gly Leu
        115                 120                 125

Pro Pro Ser Gly Ser Trp Gln Asp Leu Lys Asp His Met Arg Glu Ala
    130                 135                 140

Gly Asp Val Cys Tyr Ala Asp Val Tyr Arg Asp Gly Thr Gly Val Val
145                 150                 155                 160

Glu Phe Val Arg Lys Glu Asp Met Thr Tyr Ala Val Arg Lys Leu Asp
                165                 170                 175

Asn Thr Lys Phe Arg Ser His Glu Gly Glu Thr Ala Tyr Ile Arg Val
            180                 185                 190

Lys Val Asp Gly Pro Arg Ser Pro Ser Tyr Gly Arg Ser Arg Ser Arg
        195                 200                 205

Ser Arg Ser Arg Ser Arg Ser Arg Ser Arg Ser Asn Ser Arg Ser Arg
    210                 215                 220

Ser Tyr Ser Pro Arg Arg Ser Arg Gly Ser Pro Arg Tyr Ser Pro Arg
225                 230                 235                 240

His Ser Arg Ser Arg Ser Arg Thr
                245

<210>  4
<211>  248
<212>  PRT
<213>  Homo sapiens

<400>  4

Met Ser Gly Gly Gly Val Ile Arg Gly Pro Ala Gly Asn Asn Asp Cys
1               5                   10                  15

Arg Ile Tyr Val Gly Asn Leu Pro Pro Asp Ile Arg Thr Lys Asp Ile
            20                  25                  30

Glu Asp Val Phe Tyr Lys Tyr Gly Ala Ile Arg Asp Ile Asp Leu Lys
        35                  40                  45

Asn Arg Arg Gly Gly Pro Pro Phe Ala Phe Val Glu Phe Glu Asp Pro
    50                  55                  60

Arg Asp Ala Glu Asp Ala Val Tyr Gly Arg Asp Gly Tyr Asp Tyr Asp
65                  70                  75                  80

Gly Tyr Arg Leu Arg Val Arg Phe Pro Arg Ser Gly Arg Gly Thr Gly
                85                  90                  95

Arg Gly Gly Gly Gly Gly Gly Gly Gly Gly Ala Pro Arg Gly Arg Tyr
            100                 105                 110

Gly Pro Pro Ser Arg Arg Ser Glu Asn Arg Val Val Val Ser Gly Leu
        115                 120                 125

Pro Pro Ser Gly Ser Trp Gln Asp Leu Lys Asp His Met Arg Glu Ala
    130                 135                 140

Gly Asp Val Cys Tyr Ala Asp Val Tyr Arg Asp Gly Thr Gly Val Val
145                 150                 155                 160

Glu Phe Val Arg Lys Glu Asp Met Thr Tyr Ala Val Arg Lys Leu Asp
                165                 170                 175

Asn Thr Lys Phe Arg Ser His Glu Gly Glu Thr Ala Tyr Ile Arg Val
            180                 185                 190

Lys Val Asp Gly Pro Arg Ser Pro Ser Tyr Gly Arg Ser Arg Ser Arg
        195                 200                 205

Ser Arg Ser Arg Ser Arg Ser Arg Ser Arg Ser Asn Ser Arg Ser Arg
    210                 215                 220

Ser Tyr Ser Pro Arg Arg Ser Arg Gly Ser Pro Arg Tyr Ser Pro Arg
225                 230                 235                 240

His Ser Arg Ser Arg Ser Arg Thr
                245

<210>  5
<211>  248
<212>  PRT
<213>  Homo sapiens

<400>  5

Met Ser Gly Gly Gly Val Ile Arg Gly Pro Ala Gly Asn Asn Asp Cys
1               5                   10                  15

Arg Ile Tyr Val Gly Asn Leu Pro Pro Asp Ile Arg Thr Lys Asp Ile
            20                  25                  30

Glu Asp Val Phe Tyr Lys Tyr Gly Ala Ile Arg Asp Ile Asp Leu Lys
        35                  40                  45

Asn Arg Arg Gly Gly Pro Pro Phe Ala Phe Val Glu Phe Glu Asp Pro
    50                  55                  60

Arg Asp Ala Glu Asp Ala Val Tyr Gly Arg Asp Gly Tyr Asp Tyr Asp
65                  70                  75                  80

Gly Tyr Arg Leu Arg Val Gln Phe Pro Arg Ser Gly Arg Gly Thr Gly
                85                  90                  95

Arg Gly Gly Gly Gly Gly Gly Gly Gly Gly Ala Pro Arg Gly Arg Tyr
            100                 105                 110

Gly Pro Pro Ser Arg Arg Ser Glu Asn Arg Val Val Val Ser Gly Leu
        115                 120                 125

Pro Pro Ser Gly Ser Trp Gln Asp Leu Lys Asp His Met Arg Glu Ala
    130                 135                 140

Gly Asp Val Cys Tyr Ala Asp Val Tyr Arg Asp Gly Thr Gly Val Val
145                 150                 155                 160

Glu Phe Val Arg Lys Glu Asp Met Thr Tyr Ala Val Arg Lys Leu Asp
                165                 170                 175

Asn Thr Lys Phe Arg Ser His Glu Gly Glu Thr Ala Tyr Ile Arg Val
            180                 185                 190

Lys Val Asp Gly Pro Arg Ser Pro Ser Tyr Gly Arg Ser Arg Ser Arg
        195                 200                 205

Ser Arg Ser Arg Ser Arg Ser Arg Ser Arg Ser Asn Ser Arg Ser Arg
    210                 215                 220

Ser Tyr Ser Pro Arg Arg Ser Arg Gly Ser Pro Arg Tyr Ser Pro Arg
225                 230                 235                 240

His Ser Arg Ser Arg Ser Arg Thr
                245

<210>  6
<211>  248
<212>  PRT
<213>  Homo sapiens

<400>  6

Met Ser Gly Gly Gly Val Ile Arg Gly Pro Ala Gly Asn Asn Asp Cys
1               5                   10                  15

Arg Ile Tyr Val Gly Asn Leu Pro Pro Asp Ile Arg Thr Lys Asp Ile
            20                  25                  30

Glu Asp Val Phe Tyr Lys Tyr Gly Ala Ile Arg Asp Ile Asp Leu Lys
        35                  40                  45

Asn Arg Arg Gly Gly Pro Pro Phe Ala Phe Val Glu Phe Glu Asp Pro
    50                  55                  60

Arg Asp Ala Glu Asp Ala Val Tyr Gly Arg Asp Gly Tyr Asp Tyr Asp
65                  70                  75                  80

Gly Tyr Arg Leu Arg Val His Phe Pro Arg Ser Gly Arg Gly Thr Gly
                85                  90                  95

Arg Gly Gly Gly Gly Gly Gly Gly Gly Gly Ala Pro Arg Gly Arg Tyr
            100                 105                 110

Gly Pro Pro Ser Arg Arg Ser Glu Asn Arg Val Val Val Ser Gly Leu
        115                 120                 125

Pro Pro Ser Gly Ser Trp Gln Asp Leu Lys Asp His Met Arg Glu Ala
    130                 135                 140

Gly Asp Val Cys Tyr Ala Asp Val Tyr Arg Asp Gly Thr Gly Val Val
145                 150                 155                 160

Glu Phe Val Arg Lys Glu Asp Met Thr Tyr Ala Val Arg Lys Leu Asp
                165                 170                 175

Asn Thr Lys Phe Arg Ser His Glu Gly Glu Thr Ala Tyr Ile Arg Val
            180                 185                 190

Lys Val Asp Gly Pro Arg Ser Pro Ser Tyr Gly Arg Ser Arg Ser Arg
        195                 200                 205

Ser Arg Ser Arg Ser Arg Ser Arg Ser Arg Ser Asn Ser Arg Ser Arg
    210                 215                 220

Ser Tyr Ser Pro Arg Arg Ser Arg Gly Ser Pro Arg Tyr Ser Pro Arg
225                 230                 235                 240

His Ser Arg Ser Arg Ser Arg Thr
                245

<210> 7
<211> 1641
<212> DNA

<213>  Homo sapiens

<220>
<221>  misc_binding
<222>  (1003)..(1004)
<223>  Cytosine nucleotide +6 in exon 7

<400>  7
ccacaaatgt gggagggcga taaccactcg tagaaagcgt gagaagttac tacaagcggt      60

cctcccggcc accgtactgt tccgctccca gaagccccgg gcggcggaag tcgtcactct     120

taagaaggga cggggcccca cgctgcgcac ccgcgggttt gctatggcga tgagcagcgg     180

cggcagtggt ggcggcgtcc cggagcagga ggattccgtg ctgttccggc gcggcacagg     240

ccagagcgat gattctgaca tttgggatga tacagcactg ataaaagcat atgataaagc     300

tgtggcttca tttaagcatg ctctaaagaa tggtgacatt tgtgaaactt cgggtaaacc     360

aaaaaccaca cctaaaagaa aacctgctaa gaagaataaa agccaaaaga agaatactgc     420

agcttcctta caacagtgga aagttgggga caaatgttct gccatttggt cagaagacgg     480

ttgcatttac ccagctacca ttgcttcaat tgattttaag agagaaacct gtgttgtggt     540

ttacactgga tatggaaata gagaggagca aaatctgtcc gatctacttt ccccaatctg     600

tgaagtagct aataatatag aacaaaatgc tcaagagaat gaaaatgaaa gccaagtttc     660

aacagatgaa agtgagaact ccaggtctcc tggaaataaa tcagataaca tcaagcccaa     720

atctgctcca tggaactctt ttctccctcc accacccccc atgccagggc caagactggg     780

accaggaaag ccaggtctaa aattcaatgg cccaccaccg ccaccgccac caccaccacc     840

ccacttacta tcatgctggc tgcctccatt tccttctgga ccaccaataa ttcccccacc     900

acctcccata tgtccagatt ctcttgatga tgctgatgct ttgggaagta tgttaatttc     960

atggtacatg agtggctatc atactggcta ttatatgggt ttcagacaaa atcaaaaaga    1020

aggaaggtgc tcacattcct taaattaagg agaaatgctg gcatagagca gcactaaatg    1080

acaccactaa agaaacgatc agacagatct ggaatgtgaa gcgttataga agataactgg    1140

cctcatttct tcaaaatatc aagtgttggg aaagaaaaaa ggaagtggaa tgggtaactc    1200

ttcttgatta aaagttatgt aataaccaaa tgcaatgtga aatattttac tggactctat    1260

tttgaaaaac catctgtaaa agactggggt gggggtggga ggccagcacg gtggtgaggc    1320

agttgagaaa atttgaatgt ggattagatt ttgaatgata ttggataatt attggtaatt    1380

ttatgagctg tgagaagggt gttgtagttt ataaaagact gtcttaattt gcatacttaa    1440

gcatttagga atgaagtgtt agagtgtctt aaaatgtttc aaatggttta acaaaatgta    1500

tgtgaggcgt atgtggcaaa atgttacaga atctaactgg tggacatggc tgttcattgt    1560

actgtttttt tctatcttct atatgtttaa aagtatataa taaaaatatt taattttttt    1620

ttaaaaaaaa aaaaaaaaaa a                                              1641

<210>  8
<211>  1634
<212>  DNA
<213>  Homo sapiens

<220>
<221>  misc_binding
<222>  (1003)..(1004)
<223>  Thymidine nucleotide +6 in exon 7

<400>  8
ccacaaatgt gggagggcga taaccactcg tagaaagcgt gagaagttac tacaagcggt      60

cctcccggcc accgtactgt tccgctccca gaagccccgg gcggcggaag tcgtcactct     120

taagaaggga cggggcccca cgctgcgcac ccgcgggttt gctatggcga tgagcagcgg     180

cggcagtggt ggcggcgtcc cggagcagga ggattccgtg ctgttccggc gcggcacagg     240

ccagagcgat gattctgaca tttgggatga tacagcactg ataaaagcat atgataaagc     300

tgtggcttca tttaagcatg ctctaaagaa tggtgacatt tgtgaaactt cgggtaaacc     360

aaaaaccaca cctaaaagaa aacctgctaa gaagaataaa agccaaaaga agaatactgc     420

agcttcctta caacagtgga aagttgggga caaatgttct gccatttggt cagaagacgg     480

ttgcatttac ccagctacca ttgcttcaat tgattttaag agagaaacct gtgttgtggt     540

ttacactgga tatggaaata gagaggagca aaatctgtcc gatctacttt ccccaatctg     600

tgaagtagct aataatatag aacaaaatgc tcaagagaat gaaaatgaaa gccaagtttc     660

aacagatgaa agtgagaact ccaggtctcc tggaaataaa tcagataaca tcaagcccaa     720

atctgctcca tggaactctt ttctccctcc accacccccc atgccagggc caagactggg     780

accaggaaag ccaggtctaa aattcaatgg cccaccaccg ccaccgccac caccaccacc     840

ccacttacta tcatgctggc tgcctccatt tccttctgga ccaccaataa ttcccccacc     900

acctcccata tgtccagatt ctcttgatga tgctgatgct ttgggaagta tgttaatttc     960

atggtacatg agtggctatc atactggcta ttatatgggt tttagacaaa atcaaaaaga    1020

aggaaggtgc tcacattcct taaattaagg agaaatgctg gcatagagca gcactaaatg    1080

acaccactaa agaaacgatc agacagatct ggaatgtgaa gcgttataga agataactgg    1140

cctcatttct tcaaaatatc aagtgttggg aaagaaaaaa ggaagtggaa tgggtaactc    1200

ttcttgatta aaagttatgt aataaccaaa tgcaatgtga aatattttac tggactctat    1260

tttgaaaaac catctgtaaa agactgaggt gggggtggga ggccagcacg gtggtgaggc    1320

agttgagaaa atttgaatgt ggattagatt ttgaatgata ttggataatt attggtaatt    1380

ttatgagctg tgagaagggt gttgtagttt ataaaagact gtcttaattt gcatacttaa    1440

gcatttagga atgaagtgtt agagtgtctt aaaatgtttc aaatggttta acaaaatgta 1500

tgtgaggcgt atgtggcaaa atgttacaga atctaactgg tggacatggc tgttcattgt 1560

actgtttttt tctatcttct atatgtttaa aagtatataa taaaaatatt taattttttt 1620

ttaaattaaa aaaa 1634

## Description

Although spinal muscular atrophy (SMA) is considered to be a rare disease, it is the most common genetic cause of infant death, with an incidence of 1 in 10,000 live births. This disease is characterized by death of neuronal cells in the anterior horn of spinal cord and subsequent system-wide muscle wasting (atrophy). Three main degrees of severity were reported for patients with SMA: type I patients as "nonsitters", type II patients as "sitters", and type III patients as "walkers". The type I, also called Werdnig-Hoffman disease, is the most severe form of SMA and by far the most common, comprising nearly 60% of all newly diagnosed cases. Infants usually develop respiratory failure and do not survive beyond age 2 years. There is to date no cure for this disease.

Spinal muscular atrophy (SMA) is an autosomal recessive disease characterized by progressive muscle wasting (atrophy) and in consequence mobility impairment or even immobility. SMA is caused by the decrease of SMN (Survival of Motor Neuron) protein levels in motor neurons.

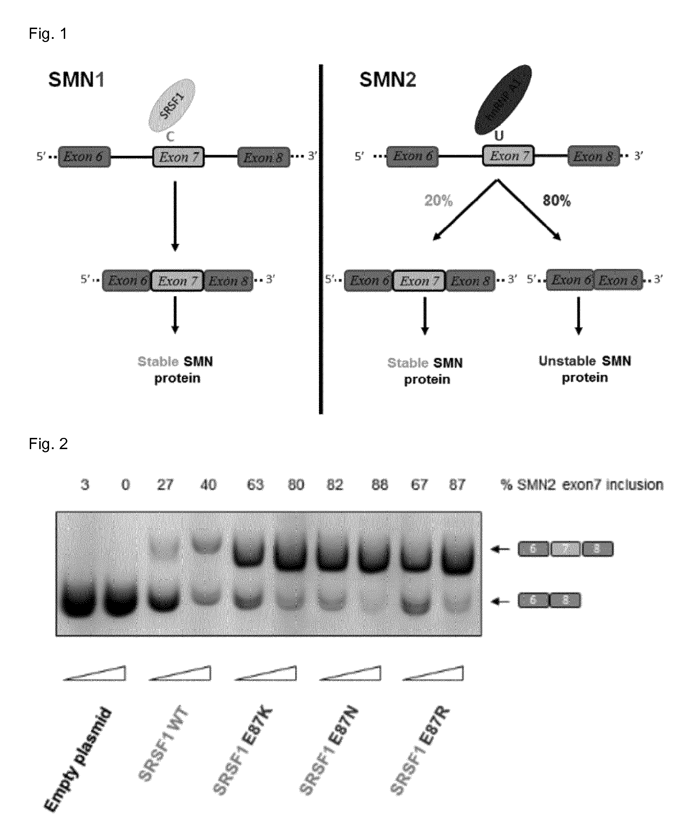

SMN protein is encoded by two genes (SMN1 and SMN2), for which the corresponding pre-mRNAs are differentially spliced (Fig. 1). Exon 7 is very efficiently included in SMN1 mRNAs, which leads to the production of functional SMN proteins. Conversely, exon 7 is primarily skipped in SMN2 mRNAs, which are translated into unstable and therefore non-functional SMN proteins. This difference in splicing is due to a single nucleotide difference at position +6 of exon 7: in SMN1, a cytosine is present and recruits the splicing activator Serine/arginine-rich splicing factor 1 (SRSF1), whereas in SMN2 a uridine is found at this position, which prevents the binding of SRSF1 and promotes instead the recruitment of the splicing repressor hnRNP A1 (Fig. 1).

In individuals affected by SMA, the SMN1 gene is mutated resulting in the expression of non-functional SMN protein. Although the usually unaffected SMN2 gene still allows the production of small amounts of SMN protein (10-20% of the normal level), this reduced availability of the SMN protein is insufficient to maintain motor neuron viability and normally results in gradual death of motor neuron cells in the anterior horn of the spinal cord and the brain. Muscles that depend on these motor neurons for neural input now have decreased innervation. These denervated muscles undergo progressive atrophy.

The problem underlying the present invention is to provide the means to increase levels of functional SMN protein to treat or prevent SMA. This problem is solved by the subject-matter of the independent claims.

The disclosed invention consists of engineering SRSF1 to force it to recognize the uridine at the position +6 of SMN2 exon 7 instead of hnRNP A1 and reverse the splicing outcome.

### Terms and definitions

Amino acid sequences are given from amino to carboxyl terminus.

*Identity* in the context of the present specification is a single quantitative parameter representing the result of a sequence comparison position by position. Methods of sequence comparison are known in the art; the BLAST algorithm available publicly is an example.

One such example for comparison of nucleic acid sequences is the BLASTN algorithm that uses the default settings: Expect threshold: 10; Word size: 28; Max matches in a query range: 0; Match/Mismatch Scores: 1.-2; Gap costs: Linear

One such example for comparison of amino acid sequences is the BLASTP algorithm that uses default settings such as: Expect threshold: 10; Word size: 3; Max matches in a query range: 0; Matrix: BLOSUM62; Gap Costs: Existence 11, Extension 1; Compositional adjustments: Conditional compositional score matrix adjustment.

Unless otherwise stated, sequence identity/similarity values provided herein refer to the value obtained using the BLAST suite of programs using default parameters detailed above.

In the context of the present specification, the term *spinal muscular atrophy (SMA)* is used in its meaning known in the art of medicine; it refers to a genetic disorder caused by a defect in the SMN1 (Survival motor neuron protein) gene as explained in greater detail above.

In the context of the present specification, the term *binding site* is used in its meaning known in the art of molecular biology; it refers to a specific nucleotide or amino acid sequence of a nucleic acid molecule or protein, which is the site of binding for a protein being able to specifically recognize this sequence.

In the context of the present specification, the term *non-naturally occurring protein* refers to artificially designed or engineered proteins that are different from naturally occurring proteins. The term thereby excludes all proteins that are natural products.

In the context of the present specification, the term *recombinant,* refers to a particular nucleic acid (DNA or RNA) that is the product of various combinations of cloning, restriction, polymerase chain reaction (PCR) and/or ligation steps resulting in a construct having a structural coding or non-coding sequence distinguishable from endogenous nucleic acids found in natural systems.

In the context of the present specification, the term *expression vector* is used in its meaning known in the art of molecular biology and genetics; it refers to a DNA construct designed to introduce a specific genetic sequence into a target cell in order to express the introduced genetic sequence. An expression vector comprises a genetic sequence to be introduced into the target cells and a promoter sequence which is operably linked to the genetic sequence.

In the context of the present specification the term *viral vector* is used in its meaning known in the art of molecular biology and genetics. It includes but is not limited to expression vectors comprising sequences of retroviruses, adenoviruses, adeno-associated viruses and herpes simplex virus.

In the context of the present specification, the terms exon and *intron* are used in their meaning known in the art of molecular biology and genetics; the term exon refers to any nucleotide sequence encoded by a gene that remains part of the final mature RNA product of that gene. The term exon refers to both the DNA sequence within a gene and to the corresponding sequence in RNA transcripts. The nucleotide sequences removed from the pre-messenger RNA by RNA splicing are referred to as introns. In RNA splicing, introns are removed and exons are covalently joined to one another as part of generating the mature messenger RNA.

In the context of the present specification, the terms *RNA splicing* or *splicing* are used in their meaning known in the art of molecular biology and genetics; they refer to a modification of pre-messenger RNA transcripts in which non-coding introns are removed and coding exons are joined. This process gives rise to mature messenger RNA (mRNA), which is translated into proteins.

In the context of the present specification, the term *splicing factor* is used in its meaning known in the art of molecular biology and genetics; it refers to a protein with the ability to regulate the splicing outcome. Splicing factors bind the pre-messenger RNA and recruit the spliceosome, which catalyse the actual splicing/cleavage and ligation reaction. However, some splicing factors can also bind the pre-messenger RNA to prevent the recruitment of the spliceosome and force an exon to be skipped from the messenger RNA instead of being ligated with the other exons.

According to a first aspect of the invention a non-naturally occurring protein is provided. The protein has an identity of ≥70%, ≥85%, ≥90%, ≥95%, or ≥99% compared to SEQ ID NO 001 (SRSF1-WT). It is characterized by the following attributes:
The protein
   i. has a splicing factor activity,
   ii. can be recruited to exon 7 of the SMN1 and/or SMN2 gene, and
   iii. facilitates the inclusion of exon 7 of the SMN1 and/or SMN2 gene into the mature SMN1 and/or SMN2 mRNA.

One example of a method known in the art to determine if a protein has the above mentioned attributes is an in vivo splicing assay as disclosed in the examples of this specification. Fig. 2 provides an example of this in vivo splicing assay. In this example the intensity of the upper band provides an indication of the efficiency of the splicing factor activity of the tested protein in including exon 7 in the resulting nucleic acid molecule. By using sequences that contain exon 7 of the SMN1 and/or SMN2 gene, one can simultaneously test if the splicing factor can be successfully recruited to this particular exon. The molecular weight of the resulting bands also provides an indication of the exons that have been joined together as depicted in Fig. 2.

One method to identify proteins with a certain percentage of sequence identity to a given sequence is the use of the BLAST algorithm. In other words proteins that are identified by using SEQ ID NO 001 in a BLAST search that fall within the identity limits set out above and are not synthetic would not be part of the first aspect of the invention.

In certain embodiments the non-naturally occurring protein of the invention is characterized in that the glutamate on position 87 of SEQ ID NO 001 is substituted by an asparagine, lysine, arginine, glutamine or histidine.

The glutamate located on position 87 in SEQ ID NO 001 might not be in the same position in other sequences that fall within the boundaries laid out in the first aspect of the invention and therefore the corresponding position for changes in the amino acid sequences has to be identified. A method to determine the corresponding position in these sequences would be the alignment of sequences. In aligned sequences the corresponding position of the glutamate on position 87 in SEQ ID NO 001 can be easily determined by comparison of the sequence aligned to position 87 of SEQ ID NO 001.

Methods of alignment of sequences for comparison are well-known in the art. Optimal alignment of sequences for comparison may be conducted by the local homology algorithm of Smith and Waterman, Adv. Appl. Math. 2:482 (1981); by the homology alignment algorithm of Needleman and Wunsch, J. Mol. Biol. 48:443 (1970); by the search for similarity method of Pearson and Lipman, Proc. Nat. Acad. Sci. 85:2444 (1988); or by computerized implementations of these algorithms.

In certain embodiments the non-naturally occurring protein is selected from SEQ ID NO 002 (E87N), SEQ ID NO 003 (E87K), SEQ ID NO 004 (E87R), SEQ ID NO 005 (E87Q) or SEQ ID NO 006 (E87H).

In certain embodiments the binding site of the non-naturally occurring protein comprises position +6 of exon 7 of the SMN1 (SEQ ID NO 007) and/or SMN2 gene (SEQ ID NO 008).

According to a second aspect of the invention a non-naturally occurring protein selected from SEQ ID NO 002 (E87N), SEQ ID NO 003 (E87K), SEQ ID NO 004 (E87R), SEQ ID NO 005 (E87Q) or SEQ ID NO 006 (E87H) is provided.

According to a third aspect of the invention a non-naturally occurring protein according to the first aspect of the invention is provided for the use as a pharmaceutical.

In certain embodiments the non-naturally occurring protein is provided for use in a treatment of spinal muscular atrophy (SMA).

In certain embodiments the nucleic acid molecule according to the fourth aspect of the invention and/or the expression vector according to the fifth aspect of the invention are provided for the use as a pharmaceutical.

According to a fourth aspect of the invention a nucleic acid molecule encoding a non-naturally occurring protein according to the first aspect of the invention is provided.

According to a fifth aspect of the invention a recombinant expression vector comprising the nucleic acid molecule according to the third aspect of the invention is provided.

In certain embodiments the nucleotide acid molecule encoding the polypeptide is operably linked to a promoter. Examples of commonly used promoters are the cytomegalovirus (CMV) and Simian virus 40 (SV40) promoters.

In certain embodiments the recombinant expression vector is a viral vector.

According to a sixth aspect of the invention a transgenic cell comprising the nucleic acid molecule according to the third aspect of the invention is provided.

According to a seventh aspect of the invention a nucleic acid molecule encoding a non-naturally occurring protein according to the first aspect of the invention for use in a treatment of spinal muscular atrophy (SMA) is provided.

According to an eighth aspect of the invention a recombinant expression vector comprising the nucleic acid molecule according to the third aspect of the invention for use in a treatment of spinal muscular atrophy (SMA) is provided.

According to a ninth aspect of the invention a transgenic cell comprising the nucleic acid molecule according to the third aspect of the invention for use in a treatment of spinal muscular atrophy (SMA) is provided.

The invention is further illustrated by the following examples and figures, from which further embodiments and advantages can be drawn. These examples are meant to illustrate the invention but not to limit its scope.

### Short description of the figures

- Fig. 1: shows effects of SRSF1 and hnRNP A1 on SMN exon 7 splicing. SRSF1 binds the cytosine (C) located at position +6 of SMN1 exon 7 and activates inclusion of the exon in the mature SMN1 mRNA, which is translated in stable and functional SMN protein. HnRNP A1 binds the uridine (U) located at the corresponding position of SMN2 exon 7 and primarily induces the skipping of the exon in the mature SMN2 mRNA, which is translated into a truncated, unstable and therefore non-functional SMN protein.
- Fig. 2: shows effects of WT and mutated versions of SRSF1 on SMN2 exon 7 splicing. HEK293 cells were cotransfected with 1 µg of pCl-SMN2 plasmid and 1 or 3 ug of the mammalian vector pCGT7 expressing WT or mutated versions of SRSF1 full length proteins. The mutated SRSF1 proteins E87K, E87N and E87R significantly increase the inclusion of exon 7 in mature SMN2 mRNAs.

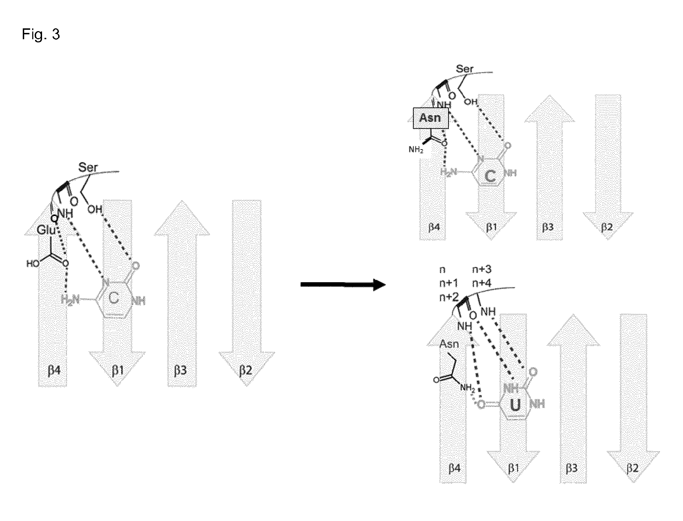

- Fig. 3: shows a schematic representation of the possible involvement of Glu87 in SRSF1 RRM1 recognition of the cytosine located at position +6 of SMN1 exon 7 (left part of the panel). In this context, the mutation of Glu87 to an asparagine residue would still allow this interaction in addition to promote the recognition of the uridine present at position +6 of SMN2 exon 7 (right part of the panel).

### Examples

Structural investigation of the splicing regulator SRSF1 revealed that the glutamate 87 located at the end of the beta4 strand of RRM1 is essential for the recognition of the cytosine located at the position +6 of SMN1 exon 7 (Figure 3). A single amino-acid substitution of this glutamate to an asparagine, lysine or arginine enlarges the initial SRSF1 binding specificity from a cytosine to the recognition of cytosine and uridine nucleotides. This mutation does not prevent the interaction of SRSF1 with its cytosine-containing natural targets in cells, while forcing its binding to the uridine located at position +6 of SMN2 exon 7. SRSF1 protein variants E87N, E87R and E87K were co-transfected in HEK293 cells with a synthetic SMN2 minigene and found that these proteins can promote up to 90% of exon 7 inclusion (Figure 2).

### Methods

### Cell culture and plasmids

Human embryonic kidney HEK293 cells were maintained in Dulbecco's modified Eagle's medium (DMEM) (GibcoBRL) supplemented with 10% fetal calf serum (GibcoBRL). These cells were cotransfected with 1 ug of pCl-SMN2 recombinant plasmid containing the SMN2 minigene (given by Stefan Stamm's lab: Department of Molecular and Cellular Biochemistry, Biomedical Biological Sciences Research Building (BBSRB), University of Kentucky, Lexington, USA) and 1 or 3 ug of the mammalian vector pCGT7 expressing WT or mutated versions of SRSF1 full length proteins. The mammalian vector expressing pCGT7::SRSF1 WT was provided by Adrian Krainer's lab (Cold Spring Harbor Laboratory, Cold Spring Harbor, NY 11724, USA) and the vectors pCGT7::SRSF1 E87N, pCGT7::SRSF1 E87K and pCGT7::SRSF1 E87R were created by site directed mutagenesis using specific primers by Dr. Antoine Cléry in Prof. Frédéric Allain's lab (ETH Zurich).

### In vivo splicing assay

HEK293 cells were plated on 6-well plates. For each transfection, 1 µg of pCl-SMN2 plasmid was cotransfected with 1 or 3 ug of the mammalian vector pCGT7 expressing WT or mutated versions (E87N, E87R, E87K) of SRSF1 full length proteins. Equal amounts of DNA were transfected by addition of empty vector when necessary. 48 hours after transfection, total RNA was isolated. 1 µg total RNA was reversed transcribed using oligo(dT) and M-MuLV Reverse Transcriptase RNAseH- (Finnzyme). 1/10 of the cDNA was amplified by PCR using a vector-specific forward primer (pCl-forw: 5'-GGTGTCCACTCCCAGTTCAA; SEQ ID NO 009) and the SMN-specific reverse primer SMNex8-rev (SMNex8-rev: 5'-GCCTCACCACCGTGCTGG; SEQ ID NO 010); 24 PCR cycles using these 3 steps: 94 °C, 30 sec; 55 °C, 30 sec; 72 °C, 60 sec. PCR products were then resolved on a 1.5% agarose gel. The ratio of exon inclusion to exon skipping was determined by using the ImageQuant software.

### Sequences:

SEQ ID NO 001 (hSRSF1): 
MSGGGVIRGPAGNNDCRIYVGNLPPDIRTKDIEDVFYKYGAIRDIDLKNRRGGPPFAFVEFEDPRDAE
DAVYGRDGYDYDGYRLRVEFPRSGRGTGRGGGGGGGGAPRGRYGPPSRRSENRVVVSGLPPSGSWQD
LKDHMREAGDVCYADVYRDGTGVVEFVRKEDMTYAVRKLDNTKFRSHEGETAYIRVKVDGPRSPSYGR
SRSRSRSRSRSRSRSRSNSRSRSYSPRRSRGSPRYSPRHSRSRSRT
SEQ ID NO 002 (E87N): 
MSGGGVIRGPAGNNDCRIYVGNLPPDIRTKDIEDVFYKYGAIRDIDLKNRRGGPPFAFVEFEDPRDAE
DAVYGRDGYDYDGYRLRVNFPRSGRGTGRGGGGGGGGAPRGRYGPPSRRSENRVVVSGLPPSGSWQD
LKDHMREAGDVCYADVYRDGTGVVEFVRKEDMTYAVRKLDNTKFRSHEGETAYIRVKVDGPRSPSYGR
SRSRSRSRSRSRSRSRSNSRSRSYSPRRSRGSPRYSPRHSRSRSRT
SEQ ID NO 003 (E87K): 
MSGGGVIRGPAGNNDCRIYVGNLPPDIRTKDIEDVFYKYGAIRDIDLKNRRGGPPFAFVEFEDPRDAE
DAVYGRDGYDYDGYRLRVKFPRSGRGTGRGGGGGGGGAPRGRYGPPSRRSENRVVVSGLPPSGSWQD
LKDHMREAGDVCYADVYRDGTGVVEFVRKEDMTYAVRKLDNTKFRSHEGETAYIRVKVDGPRSPSYGR
SRSRSRSRSRSRSRSRSNSRSRSYSPRRSRGSPRYSPRHSRSRSRT
SEQ ID NO 004 (E87R): 
MSGGGVIRGPAGNNDCRIYVGNLPPDIRTKDIEDVFYKYGAIRDIDLKNRRGGPPFAFVEFEDPRDAE
DAVYGRDGYDYDGYRLRVRFPRSGRGTGRGGGGGGGGAPRGRYGPPSRRSENRVVVSGLPPSGSWQD
LKDHMREAGDVCYADVYRDGTGVVEFVRKEDMTYAVRKLDNTKFRSHEGETAYIRVKVDGPRSPSYGR
SRSRSRSRSRSRSRSRSNSRSRSYSPRRSRGSPRYSPRHSRSRSRT
SEQ ID NO 005 (E87Q): 
MSGGGVIRGPAGNNDCRIYVGNLPPDIRTKDIEDVFYKYGAIRDIDLKNRRGGPPFAFVEFEDPRDAE
DAVYGRDGYDYDGYRLRVQFPRSGRGTGRGGGGGGGGAPRGRYGPPSRRSENRVVVSGLPPSGSWQD
LKDHMREAGDVCYADVYRDGTGVVEFVRKEDMTYAVRKLDNTKFRSHEGETAYIRVKVDGPRSPSYGR
SRSRSRSRSRSRSRSRSNSRSRSYSPRRSRGSPRYSPRHSRSRSRT
SEQ ID NO 006 (E87H): 
MSGGGVIRGPAGNNDCRIYVGNLPPDIRTKDIEDVFYKYGAIRDIDLKNRRGGPPFAFVEFEDPRDAE
DAVYGRDGYDYDGYRLRVHFPRSGRGTGRGGGGGGGGAPRGRYGPPSRRSENRVVVSGLPPSGSWQD
LKDHMREAGDVCYADVYRDGTGVVEFVRKEDMTYAVRKLDNTKFRSHEGETAYIRVKVDGPRSPSYGR
SRSRSRSRSRSRSRSRSNSRSRSYSPRRSRGSPRYSPRHSRSRSRT
SEQ ID NO 007 (hSMN1):
   >gi|196115055|ref|NM_000344.3| Homo sapiens survival of motor neuron 1, telomeric (SMN1), transcript variant d, mRNA 
CCACAAATGTGGAGGGCGATAACCACTCGTAGAAAGCGTGAGAAGTTACTACAAGCGGTCCTCCCGG
CCACCGTACTGTTCCGCTCCCAGAAGCCCCGGGCGGCGGAAGTCGTCACTCTTAAGAAGGACGGGGC
CCCACGCTGCGCACCCGCGGGTTTGCTATGGCGATGAGCAGCGGCGGCAGTGGTGGCGGCGTCCCGGA
GCAGGAGGATTCCGTGCTGTTCCGGCGCGGCACAGGCCAGAGCGATGATTCTGACATTTGGGATGATA
CAGCACTGATAAAAGCATATGATAAAGCTGTGGCTTCATTTAAGCATGCTCTAAAGAATGGTGACATT
TGTGAAACTTCGGGTAAACCAAAAACCACACCTAAAAGAAAACCTGCTAAGAAGAATAAAAGCCAAAA
GAAGAATACTGCAGCTTCCTTACAACAGTGGAAAGTTGGGGACAAATGTTCTGCCATTTGGTCAGAAG
ACGGTTGCATTTACCCAGCTACCATTGCTTCAATTGATTTTAAGAGAGAAACCTGTGTTGTGGTTTAC
ACTGGATATGGAAATAGAGAGGAGCAAAATCTGTCCGATCTACTTTCCCCAATCTGTGAAGTAGCTAA
TAATATAGAACAAAATGCTCAAGAGAATGAAAATGAAAGCCAAGTTTCAACAGATGAAAGTGAGAACT
CCAGGTCTCCTGGAAATAAATCAGATAACATCAAGCCCAAATCTGCTCCATGGAACTCTTTTCTCCCT
CCACCACCCCCCATGCCAGGGCCAAGACTGGGACCAGGAAAGCCAGGTCTAAAATTCAATGGCCCACC
 
ACCGCCACCGCCACCACCACCACCCCACTTACTATCATGCTGGCTGCCTCCATTTCCTTCTGGACCAC
CAATAATTCCCCCACCACCTCCCATATGTCCAGATTCTCTTGATGATGCTGATGCTTTGGGAAGTATG
TTAATTTCATGGTACATGAGTGGCTATCATACTGGCTATTATATGGGTTT*C*AGACAAAATCAAAAA
GAAGGAAGGTGCTCACATTCCTTAAATTAAGGAGAAATGCTGGCATAGAGCAGCACTAAATGACACCA
CTAAAGAAACGATCAGACAGATCTGGAATGTGAAGCGTTATAGAAGATAACTGGCCTCATTTCTTCAA
AATATCAAGTGTTGGGAAAGAAAAAAGGAAGTGGAATGGGTAACTCTTCTTGATTAAAAGTTATGTAA
TAACCAAATGCAATGTGAAATATTTTACTGGACTCTATTTTGAAAAACCATCTGTAAAAGACTGGGGT
GGGGGTGGGAGGCCAGCACGGTGGTGAGGCAGTTGAGAAAATTTGAATGTGGATTAGATTTTGAATGA
TATTGGATAATTATTGGTAATTTTATGAGCTGTGAGAAGGGTGTTGTAGTTTATAAAAGACTGTCTTA
ATTTGCATACTTAAGCATTTAGGAATGAAGTGTTAGAGTGTCTTAAAATGTTTCAAATGGTTTAACAA
AATGTATGTGAGGCGTATGTGGCAAAATGTTACAGAATCTAACTGGTGGACATGGCTGTTCATTGTAC
TGTTTTTTTCTATCTTCTATATGTTTAAAAGTATATAATAAAAATATTTAATTTTTTTTTAAAAAAAA
AAAAAAAAAAAA
 Nucleotide+6 in exon 7 is marked by asterisks.
SEQ ID NO 008 (hSMN2):
   >gi|196115210|ref|NM_017411.3| Homo sapiens survival of motor neuron 2, centromeric (SMN2), transcript variant d, mRNA 
CCACAAATGTGGGAGGGCGATAACCACTCGTAGAAAGCGTGAGAAGTTACTACAAGCGGTCCTCCCGG
CCACCGTACTGTTCCGCTCCCAGAAGCCCCGGGCGGCGGAAGTCGTCACTCTTAAGAAGGACGGGGC
CCCACGCTGCGCACCCGCGGGTTTGCTATGGCGATGAGCAGCGGCGGCAGTGGTGGCGGCGTCCCGGA
GCAGGAGGATTCCGTGCTGTTCCGGCGCGGCACAGGCCAGAGCGATGATTCTGACATTTGGGATGATA
CAGCACTGATAAAAGCATATGATAAAGCTGTGGCTTCATTTAAGCATGCTCTAAAGAATGGTGACATT
TGTGAAACTTCGGGTAAACCAAAAACCACACCTAAAAGAAAACCTGCTAAGAAGAATAAAAGCCAAAA
GAAGAATACTGCAGCTTCCTTACAACAGTGGAAAGTTGGGGACAAATGTTCTGCCATTTGGTCAGAAG
ACGGTTGCATTTACCCAGCTACCATTGCTTCAATTGATTTTAAGAGAGAAACCTGTGTTGTGGTTTAC
ACTGGATATGGAAATAGAGAGGAGCAAAATCTGTCCGATCTACTTTCCCCAATCTGTGAAGTAGCTAA
TAATATAGAACAAAATGCTCAAGAGAATGAAAATGAAAGCCAAGTTTCAACAGATGAAAGTGAGAACT
CCAGGTCTCCTGGAAATAAATCAGATAACATCAAGCCCAAATCTGCTCCATGGAACTCTTTTCTCCCT
CCACCACCCCCCATGCCAGGGCCAAGACTGGGACCAGGAAAGCCAGGTCTAAAATTCAATGGCCCACC
ACCGCCACCGCCACCACCACCACCCCACTTACTATCATGCTGGCTGCCTCCATTTCCTTCTGGACCAC
CAATAATTCCCCCACCACCTCCCATATGTCCAGATTCTCTTGATGATGCTGATGCTTTGGGAAGTATG
TTAATTTCATGGTACATGAGTGGCTATCATACTGGCTATTATATGGGTTT*T*AGACAAAATCAAAAA
GAAGGAAGGTGCTCACATTCCTTAAATTAAGGAGAAATGCTGGCATAGAGCAGCACTAAATGACACCA
CTAAAGAAACGATCAGACAGATCTGGAATGTGAAGCGTTATAGAAGATAACTGGCCTCATTTCTTCAA
AATATCAAGTGTTGGGAAAGAAAAAAGGAAGTGGAATGGGTAACTCTTCTTGATTAAAAGTTATGTAA
TAACCAAATGCAATGTGAAATATTTTACTGGACTCTATTTTGAAAAACCATCTGTAAAAGACTGAGGT
 
GGGGGTGGGAGGCCAGCACGGTGGTGAGGCAGTTGAGAAAATTTGAATGTGGATTAGATTTTGAATGA
TATTGGATAATTATTGGTAATTTTATGAGCTGTGAGAAGGGTGTTGTAGTTTATAAAAGACTGTCTTA
ATTTGCATACTTAAGCATTTAGGAATGAAGTGTTAGAGTGTCTTAAAATGTTTCAAATGGTTTAACAA
AATGTATGTGAGGCGTATGTGGCAAAATGTTACAGAATCTAACTGGTGGACATGGCTGTTCATTGTAC
TGTTTTTTTCTATCTTCTATATGTTTAAAAGTATATAATAAAAATATTTAATTTTTTTTTAAATTAAA
AAAA
 Nucleotide+6 in exon 7 is marked by asterisks.

## Claims

1. A non-naturally occurring protein, wherein said protein has an identity of ≥70%, ≥85%, ≥90%, ≥95%, or ≥99% compared to SEQ ID NO 001 (SRSF1-WT), **characterized in that** said protein:
i. has a splicing factor activity,
ii. can be recruited to exon 7 of the SMN1 and/or SMN2 gene, and
iii. facilitates the inclusion of said exon 7 of said SMN1 and/or SMN2 gene into the mature SMN1 and/or SMN2 mRNA.

2. The non-naturally occurring protein according to claim 1, **characterized in that** the glutamate on position 87 of SEQ ID NO 001 is substituted by an asparagine, lysine, arginine, glutamine or histidine.

3. The non-naturally occurring protein according to any one of the preceding claims, wherein said protein is selected from SEQ ID NO 002 (E87N), SEQ ID NO 003 (E87K), SEQ ID NO 004 (E87R), SEQ ID NO 005 (E87Q) or SEQ ID NO 006 (E87H).

4. A non-naturally occurring protein selected from SEQ ID NO 002 (E87N), SEQ ID NO 003 (E87K), SEQ ID NO 004 (E87R), SEQ ID NO 005 (E87Q) or SEQ ID NO 006 (E87H).

5. A non-naturally occurring protein according to any one of the preceding claims for use as a pharmaceutical.

6. A non-naturally occurring protein according to any one of claims 1 to 4 for use in a treatment of spinal muscular atrophy (SMA).

7. A nucleic acid molecule encoding a non-naturally occurring protein according to any one of the preceding claims 1 to 4.

8. A recombinant expression vector comprising the nucleic acid molecule of claim 7.

9. The recombinant expression vector of claim 8, wherein the nucleotide acid molecule encoding the polypeptide is operably linked to a promoter, particularly a promoter operable in a human cell.

10. The recombinant expression vector of claims 8 or 9, wherein the vector is a viral vector.

11. A transgenic cell comprising the nucleic acid molecule of claim 7, or the recombinant expression vector of claims 8, 9 or 10.

12. A nucleic acid molecule encoding a non-naturally occurring protein according to claim 7 for use in a treatment of spinal muscular atrophy (SMA).

13. A recombinant expression vector according to any one of claims 8 to 10 for use in a treatment of spinal muscular atrophy (SMA).

14. A transgenic cell according to claim 11 for use in a treatment of spinal muscular atrophy (SMA).
